# EUROPEAN PATENT APPLICATION

(11) **EP 4 765 032 A1**
(43) Date of publication of application: **24.06.2026**
(21) Application number: 25220200.7
(22) Date of filing: 02.12.2025
(51) Int. Cl.: G06T 19/20, G06T 17/20

(54) **IMPROVED THREE-DIMENSIONAL REPRESENTATION OF A DENTAL OBJECT**

(30) Priority: 17.12.2024 DK PA202470304
(71) Applicant: 3Shape A/S, 1060 Copenhagen K (DK)
(72) Inventor: HALLBERG, Natasha, 1060 Copenhagen K (DK); MISZTAL, Marek Krzysztof, 1060 Copenhagen K (DK); NØJGAARD, Nikolai, 1060 Copenhagen K (DK); GAWATZ, Raffael, 1060 Copenhagen K (DK)
(74) Representative: Zacco Denmark A/S

(57) **Abstract**

The present disclosure relates to a computer-implemented method and an intraoral scanning system. The computer-implemented method is configured for applying artificial interproximal tooth data to a plurality of dental objects being scanned by an intraoral scanner. The method comprising receiving intraoral scan data of a plurality of dental objects produced by an intraoral scanner, determining a 3D model of the plurality of dental objects based on the intraoral scan data, and wherein the 3D model includes a plurality of teeth vertices, segmenting, based on a tooth segmentation algorithm, the plurality of teeth vertices into a first tooth and a second tooth, and wherein an interproximal surface of the first and second tooth does not include any of the plurality of teeth vertices, fitting, based on a real-tooth fitting algorithm, a first and a second tooth model to the first and the second tooth, respectively, wherein the first and second tooth models include a plurality of reference vertices, generating artificial interproximal vertices by determining which of the plurality of reference vertices overlaps the interproximal surfaces of the first and second tooth, and applying the artificial interproximal vertices to the interproximal surface of the first and second tooth.

## Description

### FIELD

The disclosure relates to a method and an intraoral scanning system that is configured to generate an improved three-dimensional (3D) representation of a dental object. More specifically, the appearance of teeth being glued together in the 3D representation is removed.

### BACKGROUND

It is common that teeth in a 3D representation appear glued together, even though the live preview clearly shows separation between the teeth. There are two contributing factors to the gluing effect between two teeth, the first factor is due to limited resolution in the 3D representation that is used to create the scan mesh. It is effectively unable to resolve details that are smaller than three to four times a voxel resolution of around 80-150 µm in the volume that we are using for live visualization or mesh extraction. The second factor relates to systematic and statistical noise in the subscans.

US 2018/0268545 discloses how to reconstruct joined part surfaces of two adjacent teeth by repositioning nodes of the respective joined part surfaces. However, the disadvantage with the solution in the US 2018/0268545 compared to the present invention is that the joined part surfaces would not have as natural appearance since the repositioning of the nodes are not based on an artificial tooth that corresponds to the type of two adjacent teeth.

Avoiding the gluing can help to increase the perception of accuracy of the scan, but potentially also improve the local accuracy.

### SUMMARY

It is an aspect of the present disclosure to overcome the above-mentioned problem on how to avoid teeth appearing being glued together in a 3D model representation. A computer-implemented method for applying artificial interproximal tooth data to a plurality of dental objects being scanned by an intraoral scanner. The plurality of dental objects may be a dental arch of a patient that includes a plurality of teeth arranged next to each other, and the surface between the plurality of teeth is referred to as interproximal surface. For example, interproximal surface refers to the space between adjacent teeth in the direction towards the last tooth in each quadrant of a dental arch, as opposed to mesial, which refers to the direction towards the anterior midline. It is evidently that it is difficult to capture intraoral scan data that covers the interproximal surface, and therefore, to improve the visualization of the 3D model representation of the plurality of dental objects there is a need to apply the artificial interproximal tooth data to the interproximal surfaces of the dental arch. The method comprise receiving intraoral scan data of a plurality of dental objects produced by an intraoral scanner and determining a 3D model of the plurality of dental objects based on the intraoral scan data, and wherein the 3D model includes a plurality of teeth vertices.

The plurality of teeth vertices is points in space that define a 3D model's shape. Each of the plurality of teeth vertices is essentially a 3D coordinate (x, y, z) that indicates a single point on the surface of the model, e.g. 3D model of the dental object. An edge is a straight line that connects two vertices in 3D space, and a facet is a surface created by connecting multiple edges around a space. The method may further include segmenting, based on a tooth segmentation algorithm, the plurality of teeth vertices into a first tooth and a second tooth, and wherein an interproximal surface of the first and second tooth does not include any of the plurality of teeth vertices. The segmentation algorithm is configured to distinguish teeth from other elements that appear in the dental object.

The segmentation may segment the 3D model into individual teeth. This may be accompanied by identifying the teeth through a classification algorithm, e.g. PCA or a convolutional neural network. Once a tooth is identified in such a manner, it may be further segmented into buccal and lingual sides, e.g. by generating a plane between the long axis, a mesial point, and a distal point.

The segmentation may be provided by means of a computer implemented algorithm, such as a shortest path algorithm applied on a 3D matrix representing curvature of a tooth surface of the dental object.

A neural network to perform segmentation, structural/color optimization of the 3D model, removal of artificial tooth material in the 3D model, diagnostic analysis on the 3D model etc. it would be beneficial to have a computer-implemented method that is configured to generate training dataset for a neural network model. The computer-implemented method may comprise receiving a plurality of annotated digital dental 3D models of same dental object type, each annotated digital 3D dental model based on image data acquired during a scanning session and an annotation of an annotated digital dental 3D model represents a dental characteristic; identifying, for each annotated digital 3D dental model, a plurality of annotated 3D dental sub-models that are comprised in at least a part of the each annotated digital 3D model, wherein an annotated 3D dental sub-model of the plurality of annotated 3D dental sub-models partially overlaps with a neighbouring annotated 3D sub-model adjacent to the annotated 3D dental sub-model; determining a feature set corresponding to annotated 3D dental sub-models, and a classification label corresponding to the annotation of the annotated 3D dental sub-models and/ or a portion of the annotated digital 3D model corresponding to the annotated 3D dental sub-model; and generating the training dataset comprising information corresponding each of the plurality of annotated digital dental 3D models, the information comprising the determined plurality of 3D dental sub-models, feature set and classification label.

The dental object type may comprise one or more jaws, dental impressions, etc.

The scanning session may comprise a time period during which image data from a physical dental 3D object of an object type is obtained using a 3D scanning device, the time period being uninterrupted by scanning of a different physical dental 3D object by the 3D scanning device.

The annotation may comprise at least one of heat map, dental score, or a dental label.

Dental characteristics comprises at least one of diagnostic condition, tooth identification, gingiva identification, bone identification, foreign object identification (e.g. implant, veneer, rugae, restoration)

Diagnostic condition comprises at least one of tooth wear, gingival recession, caries, ......

The computer-implemented method may be configured for annotating scan patch in real-time during registration. The computer-implemented method for generating an annotated scan patch in real-time during registration may comprise receiving an input dataset comprising scan patch captured in real time during a scanning session; the input dataset through trained machine learning model and producing an output dataset comprising an annotated scan patch in real-time during registration stitched to form a 3D model.

The method may further comprise fitting, based on a real-tooth fitting algorithm, a first and a second tooth model to the first and the second tooth, respectively, and wherein the first and second tooth model include a plurality of reference vertices. The first and second tooth models are selected among a plurality of tooth models that resembles real tooth shapes. The plurality of tooth models may be created manually by human annotators, and the once that were of a poor quality have been removed. Each of the plurality of tooth models has been "normalized" in their own coordinate system such that the origin of the coordinate system is moved inside each of the plurality of tooth models. They may be subsequently reparametrized in order to have the same number of vertices as the 3D model of the dental object, e.g. the 3D model of the first and second teeth. For example, 804 or more vertices per model may be ideally.

The method may further comprise generating artificial interproximal vertices by determining which of the plurality of reference vertices overlaps the interproximal surfaces of the first and second tooth. The interproximal surfaces of the first and second tooth includes vertices that have been connected appearing as the two teeth have been melded together. These vertices are then replaced by the artificial interproximal vertices that resembles an interproximal shape of a real tooth, and thereby, the appearing of melded teeth are no more present, after the artificial interproximal vertices have been applied to the interproximal surface of the first and second tooth.

According to an aspect, an intraoral scanning system is disclosed. The intraoral scanning system may be configured to apply artificial interproximal tooth data to a plurality of dental objects being scanned by an intraoral scanner. The system may comprise an intraoral scanner configured to produce intraoral scan data that includes a plurality of dental objects, and one or more processing units. The one or more processing units may be configured to determine a 3D model of the plurality of dental objects based on the intraoral scan data, wherein the 3D model includes a plurality of teeth vertices, segment, based on a tooth segmentation algorithm, the plurality of teeth vertices into a first tooth and a second tooth, and wherein an interproximal surface of the first and second tooth does not include any of the plurality of teeth vertices, fit, based on a real-tooth fitting algorithm, a first and a second tooth model to the first and the second tooth, respectively, wherein the first and second tooth models include a plurality of reference vertices, generate artificial interproximal vertices by determine which of the plurality of reference vertices overlaps the interproximal surfaces of the first and second tooth, and apply the artificial interproximal vertices to the interproximal surface of the first and second tooth.

When applying the artificial interproximal vertices to the first and second tooth overlaps between the artificial interproximal vertices of the first and second tooth may occur. This is unwanted as that will appear artificially. The method may further comprise removing overlaps between the artificial interproximal vertices of the first tooth and the artificial interproximal vertices of the second tooth, wherein the removing of overlaps including identifying an intersection plane between the first tooth and the second tooth, wherein the first tooth and the second tooth are arranged mainly on a first side and a second side, respectively, of the intersection plane, identifying a first group of artificial interproximal vertices of the generated artificial interproximal vertices that corresponds to the first tooth which is arranged on the second side, identifying a second group of artificial interproximal vertices of the generated artificial interproximal vertices that corresponds to the second tooth which is arranged on the first side, and moving locations of the first and second group of artificial interproximal vertices to the first and second side, respectively. By moving the group of artificial interproximal vertices to the correct side of the intersection plane would result in an appearance of the interproximal surfaces of the plurality of teeth in the 3D model as being more realistic and closer to the appearance of the plurality of dental objects of the patient.

The intersection plane may be a space curve with an order of one or more branches. The neighbouring interproximal surfaces of the first and second tooth are not in parallel surfaces which means the intersection plane is a line that is in common with the first and second tooth. The intersection plane may have a longitudinal axis that is about parallel to a normal of an occlusion surface of the first and/or the second tooth.

The geometrical relations between the artificial interproximal vertices of the respective groups of artificial interproximal vertices should be maintained before and after the movement of the locations of the artificial interproximal vertices. The movement of locations of the first and second group of artificial interproximal vertices may be based on a Laplacian morph operation.

The movement of the location of the first and second group of artificial interproximal vertices may be along a first direction and a second direction, respectively, which are about perpendicular to the longitudinal axis of the intersection plane. The interproximal surface of the teeth may have a normal which the first and second direction are about parallel to. The movement of the location of the first and second group of artificial interproximal vertices is along a first direction and a second direction, respectively, that are about perpendicular to the intersection plane.

As mentioned before, the geometrical relations between the artificial interproximal vertices of the respective groups of artificial interproximal vertices should be maintained before and after the movement of the locations of the artificial interproximal vertices. The method may further comprise determining a first geometrical relation between artificial interproximal vertices of the first group of artificial interproximal vertices, determining a second geometrical relation between artificial interproximal vertices of the second group of artificial interproximal vertices, and wherein the first and second geometrical relations are maintained when the first and second group of artificial interproximal vertices are moved to the first and second side of the intersection plane, respectively.

The plurality of dental objects includes a jaw, and wherein the jaw includes a gingival and a plurality of teeth, and wherein the plurality of teeth includes at least the first tooth and the second tooth.

The 3D model may include gingival of the dental object, and the method may further include segmenting, based on a gingival segmentation algorithm, the plurality of gingival vertices into a gingival, removing the artificial interproximal vertices that overlap the plurality of gingival vertices. Some of the applied artificial interproximal vertices may overlap the gingival, and these artificial interproximal vertices should be removed.

The gingival segmentation algorithm may be configured for reconstructing a surface of the gingival based on a Poisson surface reconstruction.

Before applying the artificial interproximal vertices to the interproximal surfaces of the first and second tooth, the plurality of teeth vertices located at the interproximal surfaces may be removed based on the tooth segmentation algorithm. The plurality of teeth vertices of the two teeth that are located at opposite interproximal surfaces may form unwanted facets that have been merged appearing as the two teeth have been melded together. These vertices or facets may be removed before applying the artificial interproximal vertices. A surface of the first and second tooth may be represented by a plurality of facets, wherein corners of each of the plurality of facets corresponds to a group of vertices of the plurality of teeth vertices. The tooth segmentation algorithm may be configured for aligning the first tooth model and the second tooth model to the first tooth and the second tooth, respectively, wherein the first and second tooth models include a plurality of reference facets shaped by the plurality of reference vertices. The tooth segmentation algorithm may be further configured for comparing the plurality of facets of the first and second tooth to the plurality of reference facets of the first and second tooth models, respectively, and wherein unwanted teeth vertices of the first and second tooth corresponds to a group of vertices that represents a facet of the plurality of facets that does not match the plurality of reference facets, and removing the unwanted teeth vertices from the first and second tooth.

The interproximal surface of the first and the second tooth may not include teeth vertices, and in this example, the plurality of teeth vertices includes a plurality of boundary vertices that circumference partly the interproximal surface of the first and the second tooth, and wherein the real-tooth fitting algorithm may be configured for connecting, if a distance between the first and second boundary vertices is below a distance threshold, a first and second boundary vertices of the plurality of boundary vertices. The connection of the first and second boundary vertices would provide artificial facets that would colored according to a rending of the 3D model.

The real-tooth fitting algorithm may be configured to determine the artificial interproximal vertices. The real-tooth fitting algorithm may be configured for, fitting the first model to the first tooth by optimizing a sum of squared distances from the plurality of reference vertices of the first tooth model to the plurality of teeth vertices of the first tooth in a direction normal to the plurality of reference vertices, and fitting the second model to the second tooth by optimizing a sum of squared distances from the plurality of reference vertices of the second tooth model to the plurality of teeth vertices of the second tooth in a direction normal to the plurality of reference vertices. The real-tooth fitting algorithm may be configured for fusing the first and second model together with the first and second tooth, respectively, and determining which reference vertices of the plurality of references vertices of the first and second tooth models overlap the interproximal surface of the first and at least the second tooth, respectively, and the determined reference vertices are the artificial interproximal vertices.

To be sure that the relative positions between the teeth of the plurality of dental objects hasn't changed after the artificial interproximal vertices have been applied to the interproximal surfaces, the teeth with the artificial interproximal vertices are adjusted relative to each other based on a second 3D model. The second 3D model may be determined based on the intraoral scan data, wherein the second 3D model includes a plurality of points arranged in a point cloud, wherein the positions of the plurality of teeth vertices and the artificial interproximal vertices of the first and second tooth are adjusted relative to the plurality of points.

The method may further comprise determining a ray in a direction normal to each of the plurality of points towards each of the plurality of teeth vertices and the artificial interproximal vertices of the first and second tooth, and adjusting positions of the plurality of teeth vertices and the artificial interproximal vertices based on the ray from each of the points of the point clouds.

The method may be fully applicable for a postprocessing that may appear after the 3D model has been generated based on the intraoral scan data, or, the method may be configured to be real time processed while the 3D model is being generated or rendered. That means, while the 3D model is being generated the user may either not experience the plurality of teeth being melded together, or, may experience for a short while the melded teeth be remove in real time.

The intraoral scan data may include a plurality of subscans, and wherein the 3D model is being optimized with the artificial interproximal vertices in real time. The plurality of subscans are acquired with a frame rate of between 15 and 60 frames per second, which allows for real time rendering of the 3D model. The plurality of subscans includes intraoral scan data that are in-focus.

The 3D model includes a plurality of points arranged in a point cloud, and wherein the tooth segmentation algorithm is configured for labelling each of the plurality of points with a tooth label, and wherein the points of the plurality of points that have been labelled with the tooth label relates to the first and second tooth, removing points from the plurality of points that are not labelled with the tooth label, and segmenting the plurality of dental objects into the first and second tooth based on the labelling. Instead of segmenting the plurality of teeth into closed meshed, i.e. a tooth per closed mesh, each of the points in the point cloud are labelled such that the plurality of teeth are distinguishable from each other and gingival and other dental features being part of the plurality of dental objects.

The method may further comprise labelling each of the plurality of points with a gingival label, and wherein the points of the plurality of points that have been labelled with the gingival label relates to a gingival of the plurality of dental object, removing points from the plurality of points that are not labelled with the tooth label and gingival label, and segmenting, after the removing of points, the plurality of dental objects into a plurality of closed tooth meshes based on a tooth segmentation algorithm.

The labelling may be performed by a labelling machine learning algorithm that may be configured for receiving pixel values of the intraoral scan data and determining based on the pixel values whether a point of the plurality of points relates to a tooth or a gingival.

As mentioned, instead of labelling each of the points in the point cloud for segmenting the plurality of teeth, the tooth segmentation algorithm may be configured for segmenting the plurality of dental objects into a plurality of tooth meshes, wherein the plurality of tooth meshes includes a first and a second tooth meshes that include the first and the second tooth, respectively.

The 3D model may be rendered such that is configured for being displayed.

The one or more processing units may be configured to remove overlaps between the artificial interproximal vertices of the first tooth and the artificial interproximal vertices of the second tooth, wherein the one or more processing units may be configured to identify an intersection plane between the first tooth and the second tooth, wherein the first tooth and the second tooth are arranged mainly on a first side and a second side, respectively, of the intersection plane, identify a first group of artificial interproximal vertices of the generated artificial interproximal vertices that corresponds to the first tooth which is arranged on the second side, identify a second group of artificial interproximal vertices of the generated artificial interproximal vertices that corresponds to the second tooth which is arranged on the first side, and move locations of the first and second group of artificial interproximal vertices to the first and second side, respectively.

The one or more processing units may be configured to determine a first geometrical relation between artificial interproximal vertices of the first group of artificial interproximal vertices, determine a second geometrical relation between artificial interproximal vertices of the second group of artificial interproximal vertices, and wherein the first and second geometrical relations are maintained when the first and second group of artificial interproximal vertices are moved to the first and second side of the intersection plane, respectively.

The 3D model includes a plurality of gingival vertices, and wherein the one or more processing units may be configured to segment, based on a gingival segmentation algorithm, the plurality of gingival vertices into a gingival, and remove the artificial interproximal vertices that overlap the plurality of gingival vertices.

A surface of the first and second tooth may be represented by a plurality of facets, wherein corners of each of the plurality of facets corresponds to a group of vertices of the plurality of teeth vertices, and the tooth segmentation algorithm may be configured to align a first tooth model and a second tooth model to the first tooth and the second tooth, respectively, wherein the first and second tooth models include a plurality of reference facets shaped by the plurality of reference vertices, compare the plurality of facets of the first and second tooth to the plurality of reference facets of the first and second tooth models, respectively, and wherein unwanted teeth vertices of the first and second tooth corresponds to a group of vertices that represents a facet of the plurality of facets that does not match the plurality of reference facets, and remove the unwanted teeth vertices from the first and second tooth.

The gingival segmentation algorithm may be configured to reconstruct a surface of the gingival based on a Poisson surface reconstruction.

The plurality of teeth vertices may include a plurality of boundary vertices that circumference partly the interproximal surface of the first and the second tooth, and wherein the real-tooth fitting algorithm is configured to connect, if a distance between the first and second boundary vertices is below a distance threshold, a first and second boundary vertices of the plurality of vertices.

The real-tooth fitting algorithm may be configured to, fit, after removing unwanted teeth vertices from the first tooth, the first model to the first tooth, by optimize a sum of squared distances from the plurality of reference vertices of the first tooth model to the plurality of teeth vertices of the first tooth in a direction normal to the plurality of reference vertices, fit, after removing unwanted teeth vertices from the second tooth, the second model to the second tooth, by optimize a sum of squared distances from the plurality of reference vertices of the second tooth model to the plurality of teeth vertices of the second tooth in a direction normal to the plurality of reference vertices, fuse, after removing unwanted teeth vertices, the first and second model together with the first and second tooth, respectively, and determine reference vertices of the plurality of references vertices of the first and second tooth models that overlap the interproximal surface of the first and at least the second tooth, respectively, to be the artificial interproximal vertices.

The one or more processing units may be configured to determine a second 3D model that includes the intraoral scan data, wherein the second 3D model includes a plurality of points arranged in a point cloud and adjust positions of the plurality of teeth vertices and the artificial interproximal vertices of the first and second tooth relative to the plurality of points.

The one or more processing units may be configured to determine a ray in a direction normal to each of the plurality of points towards each of the plurality of teeth vertices and artificial interproximal vertices of the first and second tooth, and adjust positions of the plurality of teeth vertices and the artificial interproximal vertices based on the ray from each of the points of the point clouds.

The 3D model may include a plurality of points arranged in a point cloud, and wherein the tooth segmentation algorithm is configured to label each of the plurality of points with a tooth label, and wherein the points of the plurality of points that have been labelled with the tooth label relates to the first and second tooth, remove points from the plurality of points that are not labelled with the tooth label, and segment the plurality of dental objects into the first and second tooth based on the labelling.

The one or more processing units may be configured to label each of the plurality of points with a gingival label, and wherein the points of the plurality of points that have been labelled with the gingival label relates to a gingival of the plurality of dental object, remove points from the plurality of points that are not labelled with the tooth label and gingival label, and segment, after the removing of points, the plurality of dental objects into a plurality of closed tooth meshes based on a tooth segmentation algorithm.

The tooth segmentation algorithm may be configured to segment the plurality of dental objects into a plurality of tooth meshes, wherein the plurality of tooth meshes includes a first and a second tooth meshes that include the first and the second tooth, respectively.

The one or more processing units may be configured to the plurality of points by a labelling machine learning algorithm that is configured to receive pixel values of the intraoral scan data and determine based on the pixel values whether a point of the plurality of points relates to a tooth or a gingival.

The system may include a displaying unit, and wherein the one or more processing units may be configured to display the 3D model on the displaying unit, and wherein the displayed 3D model includes the first and second tooth with the artificial interproximal vertices.

The one or more processing units may be comprised by the intraoral scanner, and external computer relative to the intraoral scanner, a cloud server, and/or a server.

### BRIEF DESCRIPTION OF THE FIGURES

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 illustrates an example of a computer-implemented method,
FIGS. 2A and 2B illustrate different examples of the tooth segmentation algorithm,
FIGS. 3A, 3B, and 3C illustrate examples of removing overlaps between artificial interproximal vertices,
FIG. 4 illustrates an example of removing overlapping artificial interproximal vertices,
FIG. 5 illustrates an example of removing unwanted teeth vertices 61, and
FIGS. 6A and 6B illustrate examples on how to fill-out holes in the interproximal surfaces.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the devices, systems, mediums, programs and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A scanning for providing intra-oral scan data may be performed by a dental scanning system that may include an intraoral scanner, such as the TRIOS series scanners from 3Shape A/S. The dental scanning system may include a wireless capability as provided by a wireless network unit. The intraoral scanner may employ a scanning principle such as triangulation-based scanning, confocal scanning, focus scanning, ultrasound scanning, x-ray scanning, stereo vision, structure from motion, optical coherent tomography OCT, or any other scanning principle. In an embodiment, the scanning device is capable of obtaining surface information by operated by projecting a pattern and translating a focus plane along an optical axis of the scanning device and capturing a plurality of 2D images at different focus plane positions such that each series of captured 2D images corresponding to each focus plane forms a stack of 2D images. The acquired 2D images are also referred to herein as raw 2D images, wherein raw in this context means that the images have not been subject to image processing. The focus plane position is preferably shifted along the optical axis of the scanning system, such that 2D images captured at several focus plane positions along the optical axis form said stack of 2D images (also referred to herein as a sub-scan) for a given view of the object, i.e. for a given arrangement of the scanning system relative to the object. After moving the scanning device relative to the object or imaging the object at a different view, a new stack of 2D images for that view may be captured. The focus plane position may be varied by means of at least one focus element, e.g., a moving focus lens. The scanning device is generally moved and angled relative to the dentition during a scanning session, such that at least some sets of sub-scans overlap at least partially, to enable reconstruction of the digital dental 3D model by stitching overlapping 3D subscans together in real-time and display the progress of the virtual 3D model on a display as feedback to the user. The result of stitching is the digital 3D representation of a surface larger than that which can be captured by a single sub-scan, i.e. which is larger than the field of view of the 3D scanning device. Stitching, also known as registration and fusion, works by identifying overlapping regions of 3D surface in various sub-scans and transforming sub-scans to a common coordinate system such that the overlapping regions match, finally yielding the digital 3D model. An Iterative Closest Point (ICP) algorithm may be used for this purpose. Another example of a scanning device is a triangulation scanner, where a time varying pattern is projected onto the plurality of dental objects and a sequence of images of the different pattern configurations are acquired by one or more cameras located at an angle relative to the projector unit.

The dental scanning system may be an intraoral scanning system. The scanning device may be an intraoral scanning device.

Color texture of the plurality of dental objects may be acquired by illuminating the object using different monochromatic colors such as individual red, green and blue colors or my illuminating the object using multichromatic light such as white light. A 2D image may be acquired during a flash of white light.

Generally, the process of obtaining surface information in real time of the plurality of dental objects to be scanned requires the scanning device to illuminate the surface and acquire high number of 2D images. Typically, a high-speed camera is used with a framerate of 300-2000 2D frames pr second dependent on the technology and 2D image resolution. The high amount of image data needed to be handled by the scanning device to eighter directly forward the raw image data stream to an external processing device or performing some image processing before transmitting the data to an external device or display. This process requires that multiple electronic components inside the scanner is operating with a high workload thus requiring a high demand of current.

The scanning device comprises one or more light projectors configured to generate an illumination pattern to be projected on a three-dimensional dental object during a scanning session. The light projector(s) preferably comprises a light source, a mask signal having a spatial pattern, and one or more lenses such as collimation lenses or projection lenses. The light source may be configured to generate light of a single wavelength or a combination of wavelengths (mono- or polychromatic). The combination of wavelengths may be produced by using a light source configured to produce light (such as white light) comprising different wavelengths. Alternatively, the light projector(s) may comprise multiple light sources such as LEDs individually producing light of different wavelengths (such as red, green, and blue) that may be combined to form light comprising the different wavelengths. Thus, the light produced by the light source may be defined by a wavelength defining a specific color, or a range of different wavelengths defining a combination of colors such as white light. In an embodiment, the scanning device comprises a light source configured for exciting fluorescent material of the teeth to obtain fluorescence data from the dental object. Such a light source may be configured to produce a narrow range of wavelengths. In another embodiment, the light from the light source is infrared (IR) light, which is capable of penetrating dental tissue. The light projector(s) may be DLP projectors using a micro mirror array for generating a time varying pattern, or a diffractive optical element (DOF), or back-lit mask signal projectors, wherein the light source is placed behind a mask signal having a spatial pattern, whereby the light projected on the surface of the dental object is patterned. The back-lit mask signal projector may comprise a collimation lens for collimating the light from the light source, said collimation lens being placed between the light source and the mask signal. The mask signal may have a checkerboard pattern, such that the generated illumination pattern is a checkerboard pattern. Alternatively, the mask signal may feature other patterns such as lines or dots, etc.

The scanning device preferably further comprises optical components for directing the light from the light source to the surface of the dental object. The specific arrangement of the optical components depends on whether the scanning device is a focus scanning apparatus, a scanning device using triangulation, or any other type of scanning device. A focus scanning apparatus is further described in EP 2 442 720 B1 by the same applicant, which is incorporated herein in its entirety.

The light reflected from the dental object in response to the illumination of the dental object is directed, using optical components of the scanning device, towards the image sensor(s). The image sensor(s) are configured to generate a plurality of images based on the incoming light received from the illuminated dental object. The image sensor unit may be a high-speed image sensor such as an image sensor configured for acquiring images with exposures of less than 1/1000 second or frame rates in excess of 250 frames pr. second (fps). As an example, the image sensor may be a rolling shutter (CCD) or global shutter sensor (CMOS). The image sensor(s) may be a monochrome sensor including a color filter array such as a Bayer filter and/or additional filters that may be configured to substantially remove one or more color components from the reflected light and retain only the other non-removed components prior to conversion of the reflected light into an electrical signal. For example, such additional filters may be used to remove a certain part of a white light spectrum, such as a blue component, and retain only red and green components from a signal generated in response to exciting fluorescent material of the teeth.

The network unit may be configured to connect the dental scanning system to a network comprising a plurality of network elements including at least one network element configured to receive the processed data. The network unit may include a wireless network unit or a wired network unit. The wireless network unit is configured to wirelessly connect the dental scanning system to the network comprising the plurality of network elements including the at least one network element configured to receive the processed data. The wired network unit is configured to establish a wired connection between the dental scanning system and the network comprising the plurality of network elements including the at least one network element configured to receive the processed data.

The dental scanning system preferably further comprises a processor configured to generate scan data (such as extra-oral scan data and/or intra-oral scan data) by processing the two-dimensional (2D) images acquired by the scanning device. The processor may be part of the scanning device. As an example, the processor may comprise a Field-programmable gate array (FPGA) and/or an Advanced RISC Machines (ARM) processor located on the scanning device. The scan data comprises information relating to the three-dimensional dental object. The scan data may comprise any of: 2D images, 3D point clouds, depth data, texture data, intensity data, color data, and/or combinations thereof. As an example, the scan data may comprise one or more point clouds, wherein each point cloud comprises a set of 3D points describing the three-dimensional dental object. As another example, the scan data may comprise images, each image comprising image data e.g. described by image coordinates and a timestamp (x, y, t), wherein depth information can be inferred from the timestamp. The image sensor(s) of the scanning device may acquire a plurality of raw 2D images of the dental object in response to illuminating said object using the one or more light projectors. The plurality of raw 2D images may also be referred to herein as a stack of 2D images. The 2D images may subsequently be provided as input to the processor, which processes the 2D images to generate scan data. The processing of the 2D images may comprise the step of determining which part of each of the 2D images are in focus in order to deduce/generate depth information from the images.

The internal depth information may be used to generate 3D point clouds comprising a set of 3D points in space, e.g., described by cartesian coordinates (x, y, z). The 3D point clouds may be generated by the processor or by another processing unit. Each 2D/3D point may furthermore comprise a timestamp that indicates when the 2D/3D point was recorded, i.e., from which image in the stack of 2D images the point originates. The timestamp is correlated with the z-coordinate of the 3D points, i.e., the z-coordinate may be inferred from the timestamp. Accordingly, the output of the processor is the scan data, and the scan data may comprise image data and/or depth data, e.g. described by image coordinates and a timestamp (x, y, t) or alternatively described as (x, y, z). The scanning device may be configured to transmit other types of data in addition to the scan data. Examples of data include 3D information, texture information such as infra-red (IR) images, fluorescence images, reflectance color images, x-ray images, and/or combinations thereof.

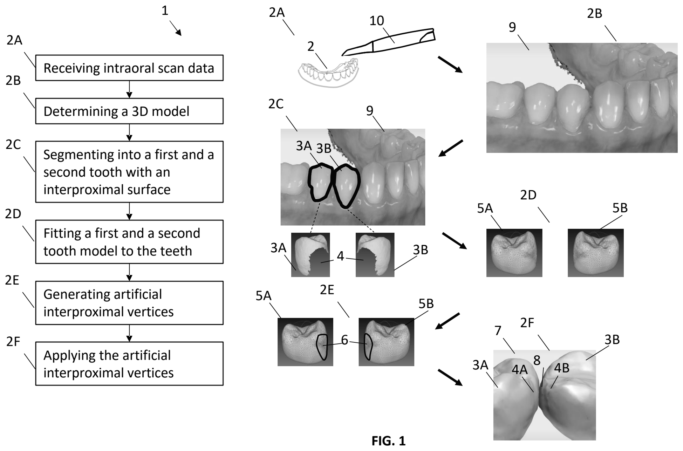

FIG. 1 illustrates a computer-implemented method 1 for applying artificial interproximal tooth data to a plurality of dental objects 2 being scanned by an intraoral scanner 10, the method 1 comprising receiving 2A intraoral scan data of a plurality of dental objects 2 produced by an intraoral scanner, and determining 2B a 3D model of the plurality of dental objects based on the intraoral scan data, and wherein the 3D model 9 includes a plurality of teeth vertices. The method 1 may further include segmenting 2C, based on a tooth segmentation algorithm, the plurality of teeth vertices into a first tooth 3A and a second tooth 3B, and wherein an interproximal surface 4 of the first and second tooth (3A,3B) does not include any of the plurality of teeth vertices. That is seen as holes 4 in the first and second tooth (3A, 3B). The method 1 may further include fitting 2D, based on a real-tooth fitting algorithm, a first 5A and a second 5B tooth model to the first 3A and the second tooth 3B, respectively, wherein the first and second tooth models (5A, 5B) include a plurality of reference vertices. The method 1 may further include generating 2E artificial interproximal vertices 6 by determining which of the plurality of reference vertices overlaps the interproximal surfaces (4A,4B) of the first and second tooth, and applying 2F the artificial interproximal vertices 6 to the interproximal surface 8 of the first and second tooth (3A, 3B). As seen, the adjacent interproximal surfaces (4A, 4B) are not melded 8 in the updated 3D model 7.

After the artificial interproximal vertices 6 have been applied to the plurality of teeth (3A, 3B) the positions and/or orientation of the plurality of teeth (3A, 3B) may have changed. The method 1 may be configured to realign the plurality of teeth of the updated 3D model 7 to the 3D model 9 that does not include the artificial interproximal vertices 6, and thereby, if any changes to the position and orientation have occurred then these will be removed due to the realignment. For example, the method 1 may include determining a second 3D model that includes the intraoral scan data, wherein the second 3D model includes a plurality of points arranged in a point cloud, and adjusting positions of the plurality of teeth vertices and the artificial interproximal vertices of the first and second tooth relative to the plurality of points. Furthermore, the method 1 may include determining a ray in a direction normal to each of the plurality of points towards each of the plurality of teeth vertices and artificial interproximal vertices of the first and second tooth, and adjusting positions of the plurality of teeth vertices and the artificial interproximal vertices based on the ray from each of the points of the point clouds.

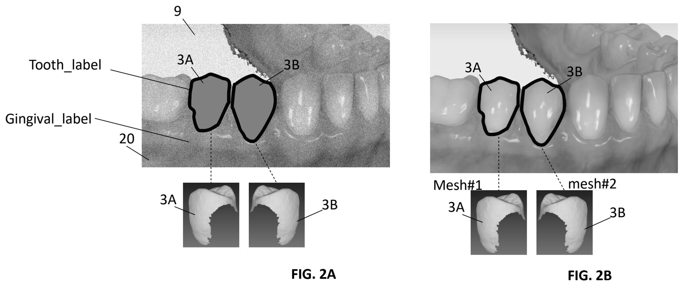

FIGS. 2A and 2B illustrate different examples of the tooth segmentation algorithm. In FIG 2A, the tooth segmentation algorithm is based on label segmentation which includes the process of classifying pixels in an image to identify distinct and meaningful regions for object recognition, such as teeth and gingival. It involves dividing the volumes into different regions that belong to a specific class, which enables tasks such as identifying teeth (3A, 3B) and gingival 20 in intraoral scan data. The tooth segmentation algorithm is configured for labelling each of the plurality of points with a tooth label, and wherein the points of the plurality of points that have been labelled with the tooth label relates to the first and second tooth, removing points from the plurality of points that are not labelled with the tooth label, and segmenting the plurality of dental objects into the first and second tooth based on the labelling.

FIG. 2B illustrates an example of the tooth segmentation algorithm that is configured for segmenting the plurality of dental objects 9 into a plurality of tooth meshes, wherein the plurality of tooth meshes includes a first and a second tooth meshes that include the first and the second tooth (3A, 3B), respectively.

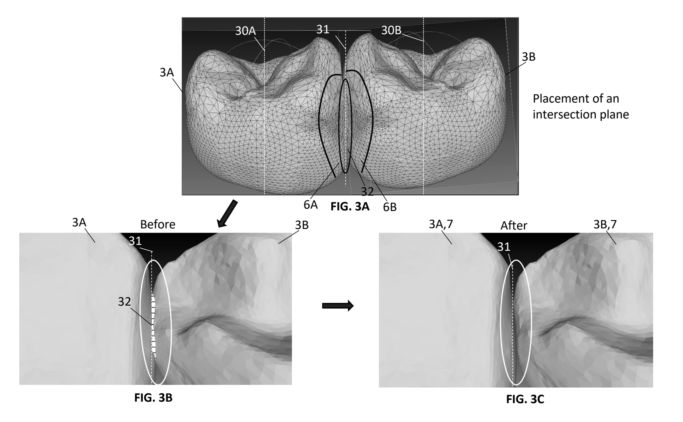

FIGS. 3A, 3B and 3C illustrate examples of removing overlaps 32 between the artificial interproximal vertices (6A, 6B) of the first and second tooth (3A,3B). In FIG. 3A, both teeth (3A, 3B) include a plurality of teeth vertices 31 and artificial interproximal vertices (6A, 6B). The method 1 is configured to remove the overlaps by, identifying an intersection plane 31 between the first tooth 3A and the second tooth 3B, wherein the first tooth 3A and the second tooth 3B are arranged mainly on a first side and a second side, respectively, of the intersection plane 31. Then, a first group of artificial interproximal vertices of the generated artificial interproximal vertices that is located on the second side, i.e. wrong side, is identified and that corresponds to the first tooth. Furthermore, a second group of artificial interproximal vertices of the generated artificial interproximal vertices that is located on the first side, i.e. wrong side, is identified corresponds and that corresponds to the second tooth. The first and second group of artificial interproximal vertices, which are located on the wrong side of the intersection plane 31, are moved to the opposite side.

In the example illustrated in FIG. 3A, the intersection plane 31 is about parallel to a normal axis (30A, 30B) of the occlusal surface of the first and second tooth (3A, 3B). In FIG. 3B, the artificial interproximal vertices 32 that are located on the wrong side of the intersection plane 31, i.e. the first and second group of artificial intersection vertices are more clearly seen. In FIG. 3C, the first and second group of artificial intersection vertices are moved to the right side of the intersection plane, meaning that a more natural appearance of the adjacent artificial interproximal surfaces are seen.

To maintain the geometrical shape between the first and second group of the artificial interproximal vertices, the movement of locations of the first and second group of artificial interproximal vertices is based on a Laplacian morph operation.

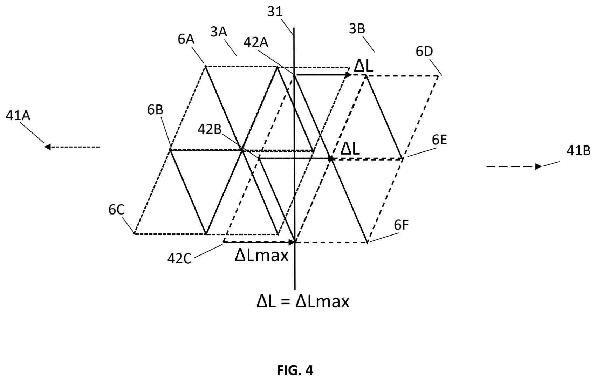

FIG. 4 illustrates an example of the artificial interproximal vertices (6A,6B,6C,6D,6E, 6F) of the first and second tooth (3A, 3B). The moving of the location of the first and second group of artificial interproximal vertices (42A, 42B, 42C) is along a first direction 41A and a second direction 41B, respectively, which are about perpendicular to the longitudinal axis of the intersection plane 31. In the example, movement of the second group of artificial interproximal vertices (42A, 42B, 42C) in the second direction 41B is illustrated. Movement of a third vertex 42C with a maximum length (ΔLmax) determines the length (ΔL) of the movement of the first and second vertices (42A, 42B). A first geometrical relation between artificial interproximal vertices of the first group of artificial interproximal vertices and a second geometrical relation between artificial interproximal vertices (42A, 42B, 42C) of the second group of artificial interproximal vertices are determined, and the first and second geometrical relations are maintained when the first and second group of artificial interproximal vertices are moved to the first and second side of the intersection plane 31, respectively.

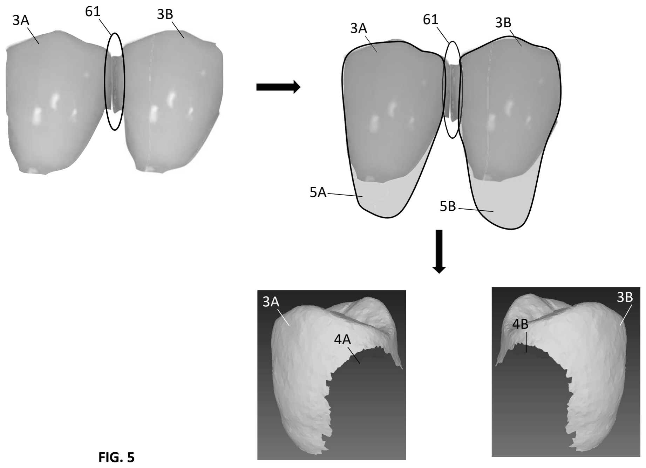

FIG. 5 illustrates an example of removing unwanted teeth vertices 61. The segmented first and second tooth (3A, 3B) from the 3D model 9 have unwanted teeth vertices 61 that were generated because of the melding effect between the two teeth (3A, 3B). These unwanted teeth vertices 61 are arranged at the interproximal surfaces (4A, 4B) of the two teeth, and the unwanted teeth vertices 61 should advantageously be removed before applying the artificial interproximal vertices 6. A surface of the first and second tooth (3A, 3B) is represented by a plurality of facets, wherein corners of each of the plurality of facets corresponds to a group of vertices of the plurality of teeth vertices. In this example, the tooth segmentation algorithm may be configured for aligning the first tooth model and the second tooth model (5A, 5B) to the first tooth 3A and the second tooth 3B, respectively, and wherein the first and second tooth models (5A, 5B) include a plurality of reference facets shaped by the plurality of reference vertices. Furthermore, the tooth segmentation algorithm may further be configured for comparing the plurality of facets of the first and second tooth (3A,3B) to the plurality of reference facets of the first and second tooth models (5A,5B), respectively, and wherein unwanted teeth vertices 61 of the first and second tooth corresponds to a group of vertices that represents a facet of the plurality of facets that does not match the plurality of reference facets, and the unwanted teeth vertices 61 are then removed leaving a hole in the inter proximal surfaces (4A, 4B) of the two teeth (3A, 3B).

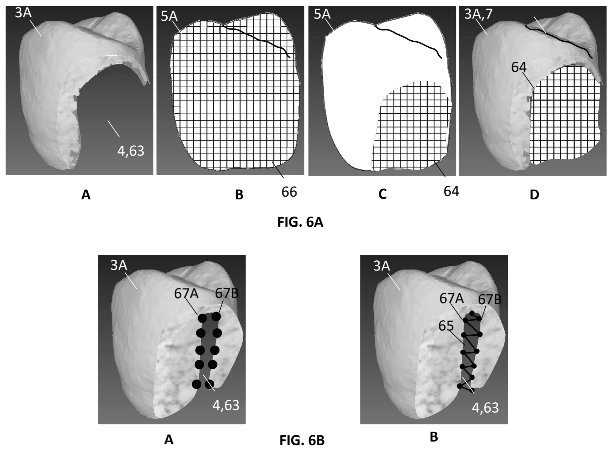

FIGS. 6A and 6B illustrate two different examples on how to fill-out the hole in the interproximal surfaces of the teeth (3A,3B) after the unwanted interproximal vertices 61 have been removed. In FIG. 6A step A, a hole 63 is seen in the interproximal surface 4 of the first tooth 3A after the unwanted artificial vertices 61 have been removed. In Step B, a first tooth model 5A with a plurality of reference vertices 66 are aligned to the first tooth 3A, and in step C, the reference vertices 64 that overlaps the hole 63 would be determined as the artificial interproximal vertices 64 that should be used for closing the hole 63 in the first tooth 3A, ass seen in step D. In more detail, the real-tooth fitting algorithm is configured for fitting the first model 5A to the first tooth 3A by optimizing a sum of squared distances from the plurality of reference vertices 66 of the first tooth model 5A to the plurality of teeth vertices of the first tooth 3A in a direction normal to the plurality of reference vertices, fusing (step C) the first model together with the first tooth, and determining which reference vertices of the plurality of references vertices 64 of the first model 5A overlap the interproximal surface 4 of the first tooth, and the determined reference vertices are the artificial interproximal vertices 64 that should be applied to close hole in the interproximal surface of the first tooth 3A.

FIG. 6B illustrates an example where the plurality of teeth vertices includes a plurality of boundary vertices (67A, 67B) that circumference partly the interproximal surface 4 of the first tooth 3A, and wherein the real-tooth fitting algorithm is configured for connecting, if a distance between the first 67A and second boundary 67B vertices is below a distance threshold, a first 67A and second boundary vertices 67B of the plurality of boundary vertices. Artificial facets 65 are then generated in the hole 4, and these artificial facets would be assign to a color during the rendering of the first
tooth 3A.

Many modifications and other embodiments of the inventions set forth herein will come to mind of one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

### ITEMS

1. A computer-implemented method for applying artificial interproximal tooth data to a plurality of dental objects being scanned by an intraoral scanner, the method comprising:
   - receiving intraoral scan data of a plurality of dental objects produced by an intraoral scanner,
   - determining a 3D model of the plurality of dental objects based on the intraoral scan data, and wherein the 3D model includes a plurality of teeth vertices,
   - segmenting, based on a tooth segmentation algorithm, the plurality of teeth vertices into a first tooth and a second tooth, and wherein an interproximal surface of the first and second tooth does not include any of the plurality of teeth vertices,
   - fitting, based on a real-tooth fitting algorithm, a first and a second tooth model to the first and the second tooth, respectively, wherein the first and second tooth models include a plurality of reference vertices,
   - generating artificial interproximal vertices by determining which of the plurality of reference vertices overlaps the interproximal surfaces of the first and second tooth, and
   - applying the artificial interproximal vertices to the interproximal surface of the first and second tooth.
2. The computer-implemented method according to item 1, comprising removing overlaps between the artificial interproximal vertices of the first tooth and the artificial interproximal vertices of the second tooth, wherein the removing of overlaps including:
   ∘ identifying an intersection plane between the first tooth and the second tooth, wherein the first tooth and the second tooth are arranged mainly on a first side and a second side, respectively, of the intersection plane,
   ∘ identifying a first group of artificial interproximal vertices of the generated artificial interproximal vertices that corresponds to the first tooth which is arranged on the second side,
   ∘ identifying a second group of artificial interproximal vertices of the generated artificial interproximal vertices that corresponds to the second tooth which is arranged on the first side, and
   ∘ moving locations of the first and second group of artificial interproximal vertices to the first and second side, respectively.
3. The computer-implemented method according to any of the previous items, wherein the intersection plane is a space curve with an order of one or more branches.
4. The computer-implemented method according to any of the previous items, wherein the intersection plane has a longitudinal axis that is about parallel to a normal of an occlusion surface of the first and/or the second tooth.
5. The computer-implemented method according to item 2, wherein the moving of locations of the first and second group of artificial interproximal vertices is based on a Laplacian morph operation.
6. The computer-implemented method according to items 2 or 5, wherein the moving of the location of the first and second group of artificial interproximal vertices is along a first direction and a second direction, respectively, which are about perpendicular to the longitudinal axis of the intersection plane.
7. The computer-implemented method according to item 2, 5 or 6, wherein the moving of the location of the first and second group of artificial interproximal vertices is along a first direction and a second direction, respectively, that are about perpendicular to the intersection plane.
8. The computer-implemented method according to any of items 2 - 7, comprising:
   - determining a first geometrical relation between artificial interproximal vertices of the first group of artificial interproximal vertices,
   - determining a second geometrical relation between artificial interproximal vertices of the second group of artificial interproximal vertices, and
   wherein the first and second geometrical relations are maintained when the first and second group of artificial interproximal vertices are moved to the first and second side of the intersection plane, respectively.
9.The computer-implemented method according to any of the previous items, wherein the 3D model includes a plurality of gingival vertices, wherein the method comprising:
   - segmenting, based on a gingival segmentation algorithm, the plurality of gingival vertices into a gingival, and
   - removing the artificial interproximal vertices that overlap the plurality of gingival vertices.
10. The computer-implemented method according to any of the previous items, wherein a surface of the first and second tooth is represented by a plurality of facets, wherein corners of each of the plurality of facets corresponds to a group of vertices of the plurality of teeth vertices, and the tooth segmentation algorithm is configured for:
   ∘ aligning the first tooth model and the second tooth model to the first tooth and the second tooth, respectively, and wherein the first and second tooth models include a plurality of reference facets shaped by the plurality of reference vertices,
   ∘ comparing the plurality of facets of the first and second tooth to the plurality of reference facets of the first and second tooth models, respectively, and wherein unwanted teeth vertices of the first and second tooth corresponds to a group of vertices that represents a facet of the plurality of facets that does not match the plurality of reference facets, and
   o removing the unwanted teeth vertices from the first and second tooth.
11. The computer-implemented method according to item 9, wherein the gingival segmentation algorithm is configured for reconstructing a surface of the gingival based on a Poisson surface reconstruction.
12. The computer-implemented method according to any of the previous items, wherein the plurality of teeth vertices includes a plurality of boundary vertices that circumference partly the interproximal surface of the first and the second tooth, and wherein the real-tooth fitting algorithm is configured for connecting, if a distance between the first and second boundary vertices is below a distance threshold, a first and second boundary vertices of the plurality of boundary vertices.
13. The computer-implemented method according to any of the precious items, wherein the real-tooth fitting algorithm is configured for,
   - fitting the first model to the first tooth by optimizing a sum of squared distances from the plurality of reference vertices of the first tooth model to the plurality of teeth vertices of the first tooth in a direction normal to the plurality of reference vertices,
   - fitting the second model to the second tooth by optimizing a sum of squared distances from the plurality of reference vertices of the second tooth model to the plurality of teeth vertices of the second tooth in a direction normal to the plurality of reference vertices,
   - fusing the first and second model together with the first and second tooth, respectively, and
   - determining which reference vertices of the plurality of references vertices of the first and second tooth models overlap the interproximal surface of the first and at least the second tooth, respectively, and the determined reference vertices are the artificial interproximal vertices.
14. The computer-implemented method according to any of the previous items, comprising
   - determining a second 3D model that includes the intraoral scan data, wherein the second 3D model includes a plurality of points arranged in a point cloud, and
   - adjusting positions of the plurality of teeth vertices and the artificial interproximal vertices of the first and second tooth relative to the plurality of points.
15. The computer-implemented method according to claim 14, comprising:
   - determining a ray in a direction normal to each of the plurality of points towards each of the plurality of teeth vertices and artificial interproximal vertices of the first and second tooth, and
   - adjusting positions of the plurality of teeth vertices and the artificial interproximal vertices based on the ray from each of the points of the point clouds.
16. The computer-implemented method according to any of the previous items, wherein the plurality of dental objects includes a jaw, and wherein the jaw includes a gingival and a plurality of teeth, and wherein the plurality of teeth includes at least the first tooth and the second tooth.
17. The computer-implemented method according to any of the items, wherein the 3D model includes a plurality of points arranged in a point cloud, and wherein the tooth segmentation algorithm is configured for:
   - labelling each of the plurality of points with a tooth label, and wherein the points of the plurality of points that have been labelled with the tooth label relates to the first and second tooth,
   - removing points from the plurality of points that are not labelled with the tooth label, and
   - segmenting the plurality of dental objects into the first and second tooth based on the labelling.
18. The computer-implemented method according to item 17, comprising:
   - labelling each of the plurality of points with a gingival label, and wherein the points of the plurality of points that have been labelled with the gingival label relates to a gingival of the plurality of dental object,
   - removing points from the plurality of points that are not labelled with the tooth label and gingival label, and
   - segmenting, after the removing of points, the plurality of dental objects into a plurality of closed tooth meshes based on the tooth segmentation algorithm.
19. The computer-implemented method according to any of items 1 to 16, wherein the tooth segmentation algorithm is configured for segmenting the plurality of dental objects into a plurality of tooth meshes, wherein the plurality of tooth meshes includes a first and a second tooth meshes that include the first and the second tooth, respectively.
20. The computer-implemented method according to any of items 17 and 18, wherein the labelling is performed by a labelling machine learning algorithm that is configured for receiving pixel values of the intraoral scan data and determining based on the pixel values whether a point of the plurality of points relates to a tooth or a gingival.
21. The computer-implemented method according to any of the previous items, comprising displaying the 3D model that includes the first and second tooth with the artificial interproximal vertices.
22. An intraoral scanning system that is configured to apply artificial interproximal tooth data to a plurality of dental objects being scanned by an intraoral scanner, wherein the system comprises:
   - an intraoral scanner configured to produce intraoral scan data that includes a plurality of dental objects,
   - one or more processing units configured to:
      ∘ determine a 3D model of the plurality of dental objects based on the intraoral scan data, wherein the 3D model includes a plurality of teeth vertices,
      ∘ segment, based on a tooth segmentation algorithm, the plurality of teeth vertices into a first tooth and a second tooth, and wherein an interproximal surface of the first and second tooth does not include any of the plurality of teeth vertices
      ∘ fit, based on a real-tooth fitting algorithm, a first and a second tooth model to the first and the second tooth, respectively, wherein the first and second tooth models include a plurality of reference vertices,
      ∘ generate artificial interproximal vertices by determine which of the plurality of reference vertices overlaps the interproximal surfaces of the first and second tooth, and
      ∘ apply the artificial interproximal vertices to the interproximal surface of the first and second tooth.
23. The intraoral scanning system according to item 22, wherein the one or more processing units is configured to remove overlaps between the artificial interproximal vertices of the first tooth and the artificial interproximal vertices of the second tooth, wherein the one or more processing units is configured to:
   ∘ identify an intersection plane between the first tooth and the second tooth, wherein the first tooth and the second tooth are arranged mainly on a first side and a second side, respectively, of the intersection plane,
   ∘ identify a first group of artificial interproximal vertices of the generated artificial interproximal vertices that corresponds to the first tooth which is arranged on the second side,
   ∘ identify a second group of artificial interproximal vertices of the generated artificial interproximal vertices that corresponds to the second tooth which is arranged on the first side, and
   ∘ move locations of the first and second group of artificial interproximal vertices to the first and second side, respectively.
24. The intraoral scanning system according to item 23, wherein the movement of locations of the first and second group of artificial interproximal vertices is based on a Laplacian morph operation.
25. The intraoral scanning system according to item 23 or 24, wherein the movement of the location of the first and second group of artificial interproximal vertices is along a first direction and a second direction, respectively, that are about perpendicular to the intersection plane.
26. The intraoral scanning system according to any of items 23 - 25, wherein the one or more processing units is configured to:
   - determine a first geometrical relation between artificial interproximal vertices of the first group of artificial interproximal vertices,
   - determine a second geometrical relation between artificial interproximal vertices of the second group of artificial interproximal vertices, and
   wherein the first and second geometrical relations are maintained when the first and second group of artificial interproximal vertices are moved to the first and second side of the intersection plane, respectively.
27.The intraoral scanning system according to any of items 22 to 26 wherein the 3D model includes a plurality of gingival vertices, and wherein the one or more processing units is configured to:
   - segment, based on a gingival segmentation algorithm, the plurality of gingival vertices into a gingival, and
   - remove the artificial interproximal vertices that overlap the plurality of gingival vertices.
28. The intraoral scanning system according to any of items 22 - 27, wherein a surface of the first and second tooth is represented by a plurality of facets, wherein corners of each of the plurality of facets corresponds to a group of vertices of the plurality of teeth vertices, and the tooth segmentation algorithm is configured to:
   ∘ align a first tooth model and a second tooth model to the first tooth and the second tooth, respectively, wherein the first and second tooth models include a plurality of reference facets shaped by the plurality of reference vertices,
   ∘ compare the plurality of facets of the first and second tooth to the plurality of reference facets of the first and second tooth models, respectively, and wherein unwanted teeth vertices of the first and second tooth corresponds to a group of vertices that represents a facet of the plurality of facets that does not match the plurality of reference facets, and
   o remove the unwanted teeth vertices from the first and second tooth.
29. The intraoral scanning system according to item 27, wherein the gingival segmentation algorithm is configured to reconstruct a surface of the gingival based on a Poisson surface reconstruction.
30. The intraoral scanning system according to any of items 22 to 29, wherein the plurality of teeth vertices includes a plurality of boundary vertices that circumference partly the interproximal surface of the first and the second tooth, and wherein the real-tooth fitting algorithm is configured to connect, if a distance between the first and second boundary vertices is below a distance threshold, a first and second boundary vertices of the plurality of vertices.
31. The intraoral scanning system according to any of items 22 to 30, wherein the real-tooth fitting algorithm is configured to,
   - fit, after removing unwanted teeth vertices from the first tooth, the first model to the first tooth, by optimize a sum of squared distances from the plurality of reference vertices of the first tooth model to the plurality of teeth vertices of the first tooth in a direction normal to the plurality of reference vertices,
   - fit, after removing unwanted teeth vertices from the second tooth, the second model to the second tooth, by optimize a sum of squared distances from the plurality of reference vertices of the second tooth model to the plurality of teeth vertices of the second tooth in a direction normal to the plurality of reference vertices,
   - fuse, after removing unwanted teeth vertices, the first and second model together with the first and second tooth, respectively, and
   - determine reference vertices of the plurality of references vertices of the first and second tooth models that overlap the interproximal surface of the first and at least the second tooth, respectively, to be the artificial interproximal vertices.
32. The intraoral scanning system according to any of items 22 - 31, wherein the one or more processing units is configured to:
   - determine a second 3D model that includes the intraoral scan data, wherein the second 3D model includes a plurality of points arranged in a point cloud, and
   - adjust positions of the plurality of teeth vertices and the artificial interproximal vertices of the first and second tooth relative to the plurality of points.
33. The intraoral scanning system according to item 32, wherein the one or more processing units is configured to:
   - determine a ray in a direction normal to each of the plurality of points towards each of the plurality of teeth vertices and artificial interproximal vertices of the first and second tooth, and
   - adjust positions of the plurality of teeth vertices and the artificial interproximal vertices based on the ray from each of the points of the point clouds.
34. The intraoral scanning system according to any of items 22 to 33, wherein the plurality of dental objects includes a jaw, and wherein the jaw includes a gingival and a plurality of teeth, and wherein the plurality of teeth includes at least the first tooth and the second tooth.
35. The intraoral scanning system according to any of items 22 to 34, wherein the intraoral scan data is a subscan that includes the plurality of dental objects, and wherein the artificial interproximal vertices are applied to the first and second tooth in real time.
36. The intraoral scanning system according to any of 22 to 36, wherein the 3D model includes a plurality of points arranged in a point cloud, and wherein the tooth segmentation algorithm is configured to:
   - label each of the plurality of points with a tooth label, and wherein the points of the plurality of points that have been labelled with the tooth label relates to the first and second tooth,
   - remove points from the plurality of points that are not labelled with the tooth label, and
   - segment the plurality of dental objects into the first and second tooth based on the labelling.
37. The intraoral scanning system according to item 36, wherein the one or more processing units is configured to:
   - label each of the plurality of points with a gingival label, and wherein the points of the plurality of points that have been labelled with the gingival label relates to a gingival of the plurality of dental object,
   - remove points from the plurality of points that are not labelled with the tooth label and gingival label, and
   - segment, after the removing of points, the plurality of dental objects into a plurality of closed tooth meshes based on a tooth segmentation algorithm.
38. The intraoral scanning system according to any of items 22 to 35, wherein the tooth segmentation algorithm is configured to segment the plurality of dental objects into a plurality of tooth meshes, wherein the plurality of tooth meshes includes a first and a second tooth meshes that include the first and the second tooth, respectively.
39. The intraoral scanning system according to any of items 36 and 37, wherein the one or more processing units is configured to the plurality of points by a labelling machine learning algorithm that is configured to receive pixel values of the intraoral scan data and determine based on the pixel values whether a point of the plurality of points relates to a tooth or a gingival.
40. The intraoral scanning system according to any of items 22 to 39, comprised a displaying unit, and wherein the one or more processing units is configured to display the 3D model on the displaying unit, and wherein the displayed 3D model includes the first and second tooth with the artificial interproximal vertices.

## Claims

1. A computer-implemented method for applying artificial interproximal tooth data to a plurality of dental objects being scanned by an intraoral scanner, the method comprising:
• receiving intraoral scan data of a plurality of dental objects produced by an intraoral scanner,
• determining a 3D model of the plurality of dental objects based on the intraoral scan data, and wherein the 3D model includes a plurality of teeth vertices,
• segmenting, based on a tooth segmentation algorithm, the plurality of teeth vertices into a first tooth and a second tooth, and wherein an interproximal surface of the first and second tooth does not include any of the plurality of teeth vertices,
• fitting, based on a real-tooth fitting algorithm, a first and a second tooth model to the first and the second tooth, respectively, wherein the first and second tooth models include a plurality of reference vertices,
• generating artificial interproximal vertices by determining which of the plurality of reference vertices overlaps the interproximal surfaces of the first and second tooth, and
• applying the artificial interproximal vertices to the interproximal surface of the first and second tooth.

2. The computer-implemented method according to claim 1, comprising removing overlaps between the artificial interproximal vertices of the first tooth and the artificial interproximal vertices of the second tooth, wherein the removing of overlaps including:
∘ identifying an intersection plane between the first tooth and the second tooth, wherein the first tooth and the second tooth are arranged mainly on a first side and a second side, respectively, of the intersection plane,
∘ identifying a first group of artificial interproximal vertices of the generated artificial interproximal vertices that corresponds to the first tooth which is arranged on the second side,
∘ identifying a second group of artificial interproximal vertices of the generated artificial interproximal vertices that corresponds to the second tooth which is arranged on the first side, and
∘ moving locations of the first and second group of artificial interproximal vertices to the first and second side, respectively.

3. The computer-implemented method according to any of the previous claims, wherein the intersection plane is a space curve with an order of one or more branches.

4. The computer-implemented method according to any of the previous claims, wherein the intersection plane has a longitudinal axis that is about parallel to a normal of an occlusion surface of the first and/or the second tooth.

5. The computer-implemented method according to claim 2, wherein the moving of locations of the first and second group of artificial interproximal vertices is based on a Laplacian morph operation.

6. The computer-implemented method according to claims 2 or 5, wherein the moving of the location of the first and second group of artificial interproximal vertices is along a first direction and a second direction, respectively, which are about perpendicular to the longitudinal axis of the intersection plane.

7. The computer-implemented method according to claim 2, 5 or 6, wherein the moving of the location of the first and second group of artificial interproximal vertices is along a first direction and a second direction, respectively, that are about perpendicular to the intersection plane.

8. The computer-implemented method according to any of claims 2 - 7, comprising:
• determining a first geometrical relation between artificial interproximal vertices of the first group of artificial interproximal vertices,
• determining a second geometrical relation between artificial interproximal vertices of the second group of artificial interproximal vertices, and
wherein the first and second geometrical relations are maintained when the first and second group of artificial interproximal vertices are moved to the first and second side of the intersection plane, respectively.

9. The computer-implemented method according to any of the previous claims, wherein the 3D model includes a plurality of gingival vertices, wherein the method comprising:
• segmenting, based on a gingival segmentation algorithm, the plurality of gingival vertices into a gingival, and
• removing the artificial interproximal vertices that overlap the plurality of gingival vertices.

10. The computer-implemented method according to any of the previous claims, wherein a surface of the first and second tooth is represented by a plurality of facets, wherein corners of each of the plurality of facets corresponds to a group of vertices of the plurality of teeth vertices, and the tooth segmentation algorithm is configured for:
∘ aligning the first tooth model and the second tooth model to the first tooth and the second tooth, respectively, and wherein the first and second tooth models include a plurality of reference facets shaped by the plurality of reference vertices,
∘ comparing the plurality of facets of the first and second tooth to the plurality of reference facets of the first and second tooth models, respectively, and wherein unwanted teeth vertices of the first and second tooth corresponds to a group of vertices that represents a facet of the plurality of facets that does not match the plurality of reference facets, and
∘ removing the unwanted teeth vertices from the first and second tooth.

11. The computer-implemented method according to item 9, wherein the gingival segmentation algorithm is configured for reconstructing a surface of the gingival based on a Poisson surface reconstruction.

12. The computer-implemented method according to any of the previous items, wherein the plurality of teeth vertices includes a plurality of boundary vertices that circumference partly the interproximal surface of the first and the second tooth, and wherein the real-tooth fitting algorithm is configured for connecting, if a distance between the first and second boundary vertices is below a distance threshold, a first and second boundary vertices of the plurality of boundary vertices.

13. The computer-implemented method according to any of the precious items, wherein the real-tooth fitting algorithm is configured for,
• fitting the first model to the first tooth by optimizing a sum of squared distances from the plurality of reference vertices of the first tooth model to the plurality of teeth vertices of the first tooth in a direction normal to the plurality of reference vertices,
• fitting the second model to the second tooth by optimizing a sum of squared distances from the plurality of reference vertices of the second tooth model to the plurality of teeth vertices of the second tooth in a direction normal to the plurality of reference vertices,
• fusing the first and second model together with the first and second tooth, respectively, and
• determining which reference vertices of the plurality of references vertices of the first and second tooth models overlap the interproximal surface of the first and at least the second tooth, respectively, and the determined reference vertices are the artificial interproximal vertices.

14. The computer-implemented method according to any of the previous items, comprising
• determining a second 3D model that includes the intraoral scan data, wherein the second 3D model includes a plurality of points arranged in a point cloud, and
• adjusting positions of the plurality of teeth vertices and the artificial interproximal vertices of the first and second tooth relative to the plurality of points.

15. The computer-implemented method according to claim 14, comprising:
• determining a ray in a direction normal to each of the plurality of points towards each of the plurality of teeth vertices and artificial interproximal vertices of the first and second tooth, and
• adjusting positions of the plurality of teeth vertices and the artificial interproximal vertices based on the ray from each of the points of the point clouds.
